# EUROPEAN PATENT SPECIFICATION

(11) **EP 0 679 406 B1**
(45) Date of publication and mention of the grant of the patent: **31.07.2002**
(21) Application number: 95201022.1
(22) Date of filing: 24.04.1995
(51) Int. Cl.: A61L 2/06, A61C 19/00, B08B 3/08, B08B 3/00

(54) **Method and apparatus for cleaning medical, paramedical and dental equipment and dental lines.**
Verfahren und Einrichtung zum Reinigen von medizinischen, paramedizinischen und Zahnärztlichen Geräten und Leitungen
Méthode et dispositif de nettoyage des instruments médicaux, paramédicaux et dentaires et de leur conduites

(30) Priority: 27.04.1994 NL 9400677
(43) Date of publication of application: 02.11.1995
(73) Proprietor: Nederlandse Maatschappij tot Bevordering der Tandheelkunde, NL-3435 CA Nieuwegein (NL)
(72) Inventor: Berendsen, Johannes Antonius Theodorus, NL-2361 LK Warmond (NL); Rietmeijer, Antonius George Maria, NL-1251 HP Laren (NL); Borstlap, Adrianus Johannes, NL-1217 LZ Hilversum (NL)
(74) Representative: Ferguson, Alexander

(56) References cited:
- EP-A- 0 300 945
- EP-A- 0 580 569
- FR-A- 2 098 972
- GB-A- 2 136 911
- US-A- 4 414 037
- US-A- 5 184 571

## Description

The invention relates to a method and apparatus as described in the preambles of claims 1 and 13, respectively. Such a method and such an apparatus are known from EP-A-0.300.945

On of the most important kinds of equipment which is used by dentists are angle pieces. Nowadays these angle pieces can be driven in an electrical/mechanical or in a pneumatic/mechanical way. The electrically/mechanically and pneumatically/mechanically driven angle pieces are driven by a micromotor, which is in front of the grip portion of the angle piece. The micro motor can be driven by means of electricity or compressed air. In this arrangement a driving shaft extends through the hollow spaces of the grip of the angle piece, which shaft drives a pinion near the drilling end. In the case of pneumatically driven angle pieces supply and discharge lines for driving air are accommodated in the grip portion of the angle piece in such a way that they lead the driving air past a turbine for the drill situated in the angle piece head. All angle pieces can be provided with lines for spray water and spray air which extend through the grip portion and the angle piece head.

Hand pieces are substantially straight and are especially used for dental surgical purposes. They have a cutter or drill which can be driven in a manner similar to the angle pieces.

It has been found that during use in dental practice the angle pieces are contaminated with anorganic and organic material originating from the patient's mouth, such as saliva, blood, micro-organisms, remainders of mucous membrane, drilled loose tooth and filling material, but also food particles. This seems especially to occur with pneumatically-mechanically driven angle pieces. Every time the angle piece is turned off, which for reasons of security always takes place when the angle piece head is still in the patient's mouth, the pressure in the angle piece head sinks below the atmospheric pressure, so that air and particles are sucked up from the direct surroundings of the angle piece head into the angle piece. This is intensified even more if the unit is provided with a system for active retrosuction of the spray water. These particles are in part discharged via the compressed air-return line to the unit or remain behind in the water and air line, in the angle piece, and come, on the other hand, into contact with the lubricant present the angle piece head. Possible valves do not prevent in a reliable way the discharge of the particles in lines because on account of impurities they will not always be able to work perfectly.

When restarting the piece of equipment the impurities which have got into the lines and the head will be led into the following patient's mouth. In this way exchange of micro-organisms occurs (cross-infection). These impurities form a risk for the health of subsequent patients.

In order to adequately reduce this risk it is desirable to sterilize the angle pieces in an autoclave (to autoclave). In a closed cupboard the angle pieces are hereby subjected to an environment with steam of approximately 134°C. In conventional autoclaves where steam is introduced in the cupboard space, inclusions of air are formed in the spaces of the angle pieces which are not easily accessible, where the steam cannot penetrate and the sterilizations process cannot thus be effective. Attempts have been made to improve on this by using vacuum autoclaves. Sterilisation is not guaranteed with vacuum autoclaves either, however, as the presence of lubricant in the angle piece has a protective effect on the micro-organisms present there and has a inhibiting effect on the penetration of steam introduced into the closed space, as a result of which the destroying temperature is considerably higher.

One cleaning method is the one described in EP-A-0300945 in which use is made of a closable cupboard in which there are connecting points for dental equipment. There is one connection for every connecting point comprising three lines, namely one for water, possibly in the form of mist, one for disinfectant, also possibly in the form of mist and one for lubricating oil. A possibly pulsating air flow can be fed between washing and disinfecting and between disinfecting and lubricating. The chemical disinfectants used here are, however, not permitted in all countries. Their effectiveness can be reduced, for example because of oxidation, whereby the result of the process is not constant. On safety and environment grounds objections can also be raised against the use of chemical disinfectants.

With an arrangement in which a provision is linked into the supply and discharge lines of the angle piece with the help of which it is ensured that also during turning off of the angle piece a positive pressure difference is present in the angle piece head, a (partially) preventive solution is aimed at. Here it is still necessary to sterilize the angle piece for further use.

Another problem which occurs with equipment that is at the disposal of the dentist is that of the formation of biofilm in the air and water transporting lines, in particular lines which lead to a water sprayer or to a multi-functional squirt. These lines have a small cross section and the water consumption is small (1 to 2 litres per day) and the periods of standstill are prolonged as a result of which the formation of biofilm occurs. Because of the micro-organisms present therein this biofilm formation is also a risk for the patient's health, into whose mouth water is sprayed, via a line on the inner wall of which biofilm has been formed. This problem does not only occur in the supply hoses for water but also in the water line portions which are present in the angle pieces and in the unit.

Tests have shown that spray water can contain pathogens such as Pseudomonas cepacia, Klebsiella, Staphylocuccus Saprophyticus, Pasteuralla hemolytica and Legionella, which can cause illnesses and even disturbances in the immune system. In connection with this bronchial disorders, liver infections and tuberculosis can be considered.

US-A-4.414.037 discloses a steam jet cleaning system for use in -amongst others- dental laboratories. The known system includes a nozzle by means of which a high pressurized spray of steam is forced onto an object to be cleaned which is held at a distance from the nozzle.

It is an object of the invention to provide a method and an apparatus with which keeping medical, paramedical and dental equipment having driven parts, such as angle pieces, supply hoses and units clean is facilitated and simplified, however in a safer and more reliable way. To this end, the invention provides a method according to claim 1.

In a general approach according to the invention an angle piece is subjected to a wet heat-disinfecting step in which the hollow space of the part concerned is flushed with steam. Steam administered in such a way has a very constant disinfecting capacity and can penetrate all portions of the space.

The piece of equipment is subjected to a flushing with a detergent solution prior to the steam step in order to enlarge the effect of the steam flow through. By the detergent solution first of all coagulation is prevented, proteins are denatured, the lubricating oil is emulsified and the surface tension thereof is lowered. Flushing out with detergent also has a reducing effect on the spore elements. The small number which may still be left behind can be further reduced by a longer steam through period, possibly even to nil. The small number which may still be left behind does not, however, form any risk at all in a non-invasive dental treatment because there are already several million micro-organisms in the human mouth cavity. In some types of angle pieces which contain interference fits, a better cleaning result can be obtained if the outer side of the instrument is heated at the fit from the outside by means of an adjustable steam sprayer.

It is further advantageous after flushing through with detergent solution to flush the hollow space in the piece of equipment with water to remove detergent residue.

Preferably the detergent solution is heated after having preferably been forced unheated through the hollow spaces. When treating with unheated detergent solution the proteins are broken down so that they cannot coagulate during the treatment with the heated detergent solution. Whilst flowing the temperature of the detergent solution is preferably gradually increased from room temperature to at least approximately 60°C, and then kept at that temperature for some time. It was shown during trials that with pneumatically driven angle pieces favourable results are attained if the warming up phase takes 3 minutes and the 60°C phase approximately 2 minutes, the steam flow period lasting about 5 minutes.

Even better results are attained if the detergent solution jet is lead through the hollow spaces in a pulsating way. Because of the pressure variations (for example 10 seconds flushing through and 10 seconds rest) the penetration of the detergent solution to places which are difficult to reach, such as small corners, is stimulated.

Steam flowing through the hollow spaces or lumen is done preferably in a pulsating way or intermittently so that the steam will also be active in the places most difficult to reach.

From tests it has been shown that passing the steam flow through the hollow spaces, in particular after treatment with the detergent solution, brings about a disinfection by wet heat (saturated steam) and cross-infection is thus ruled out, without it being necessary to autoclave or to apply other, chemically disinfecting means. Thus the cleaning is carried out with relatively gentle means, which do not, moreover, make any particular demands on or require attention from the user, and can be carried out in a short period.

The whole cleaning/disinfecting process can be accelerated by discharging condensation and residual heat by means of flushing through compressed air which is free of micro-organisms. In addition, the inside of the angle piece is cooled by the air before the external parts, whereby detrimental temperature stresses are prevented.

For carrying out the method according to the invention a simple apparatus is provided as described in claim 13, which is preferably provided with several connecting couplings so that several angle pieces and/or hoses can be attached thereto for cleaning purposes.

The apparatus can be mobile, so that it can be moved to the so-called treating unit, to subsequently, via short vapour-tight connected hoses, see to disinfection of the unit treating unit = the assembly of provisions for the purpose of driving and controlling dental equipment such as hand pieces and angle pieces with micromotor or turbine motor and a multi-functional squirt, either combined with a dentist's chair or not. In this way the steam can retain its effectiveness whilst only a few special provisions are needed in the unit, such as connecting coupling(s) to the various lines, and a provision (known pers se) with which the valves in the unit can be opened, and care has to be taken that the lines and connections in the unit can withstand the prevailing temperatures and steam pressure.

Further preferred embodiments of the apparatus according to the invention are described in the subclaims, which should be considered inserted here.

The invention will now be described in more detail on the basis of an exemplary embodiment shown in the accompanying drawing.
Figure 1 shows a perspective view of a mobile apparatus according to the invention, suitable for carrying out the method according to the invention;
figure 2 shows a schematic view of the relationship between the most important parts of the apparatus of figure 1, on a mutually distorted scale;
figure 3 shows a cross section of a pneumatically driven angle piece;
figure 4 shows a cross section of a mechanically driven angle piece; and
figure 5 shows a schematic view of an alternative arrangement according to the invention.

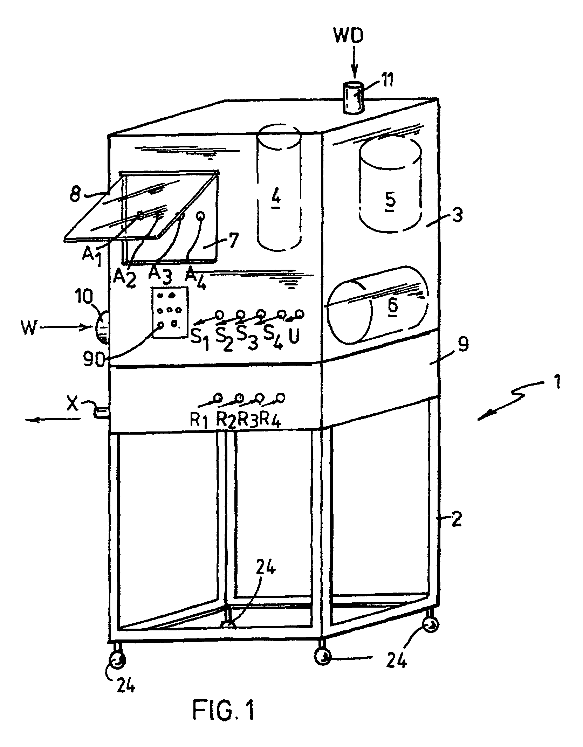

The mobile apparatus 1 shown in figure 1 comprises a frame 2, which is supported on wheels 4 to be mobile between a position where the cleaning of equipment generally takes place (outside the working area of the dentist), and a position up to near the unit, on the dentist's chair or not. On top of the frame 2 there is a cupboard 3, in which the most important parts of the apparatus according to the invention are accommodated. A reservoir 4 for tap water, a steam source 5 and a compressor 6 or a connection for compressed air are shown schematically. The steam source 5 can be filled via supply 11 with distilled or demineralized water WD and the reservoir 4 can be filled with tap water W via supply 10, possibly by means of a self-tapping connection. Suitable hoses can be connected to the closable supplies 10 and 11, if refilling is necessary. At the front of the cupboard 3 there is a bowl 7, which can be closed fluid-tight with the help of a lockable transparent lid 8, which bowl is (here) provided on the rear wall with at least four connecting couplings A₁, A₂, A₃ and A₄, ...., Aₙ, to which conventional couplings on dentist's equipment can be placed. The number of connecting possibilities is adjusted to the amount of equipment which a dentist usually uses during a treatment. Three pneumatically driven angle pieces and one micro motor angle piece can be considered. Also medical, paramedical and dental equipment is possible.

On the outside, at the front of the cupboard 3 there are another four closable connecting conplings S₁, S₂, S₃, .... Sₙ, to which the (detached) hoses which lead from the unit to the equipment can be connected. Closable connecting couplings R₁, R₂, R₃, ... Rₙ are arranged on the receptacle 9, so that the fluids brought into the hoses via S₁-Sₙ can reach the receptacle in order to be discharged later on. At the front a further closable connecting coupling U is provided, to which a (short) hose which extends to the unit or connecting cupboard can be connected. This hose is connected to the unit or connecting cupboard by means of an additional vapour-tight connecting coupling. During the flow through of the unit the equipment hoses can be connected to the couplings R₁-Rₙ. In addition, there is a control panel 90 at the front for activating the various parts.

It will be understood that the connecting couplings and the lines leading to them in the cupboard 3 are adjusted to the lines and equipment to be connected thereto.

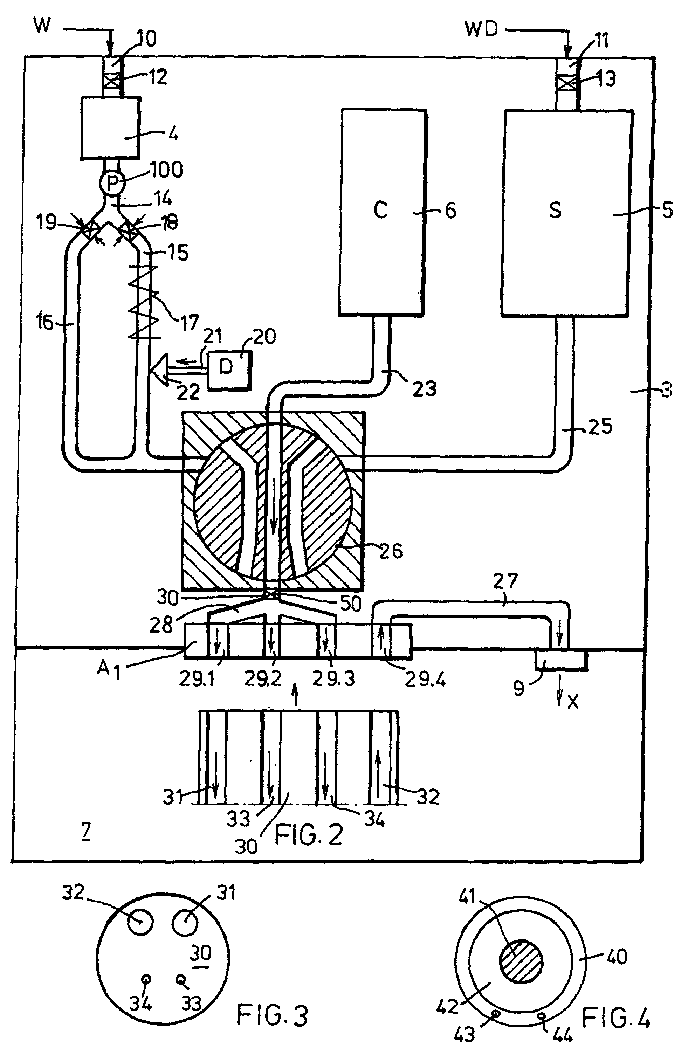

As is shown schematically in figure 2 a water reservoir 4 is accommodated in the cupboard 3 which is to be filled with water from the water mains via closable supply opening 10 and line 12, and which can deliver water via discharge line 14 because of the action of the roller pump 100. Discharge line 14 branches into lines 15 and 16, which can be closed in a selective way by means of pinch valves 18, 19, respectively, or other valves acting on the outside of the hose, which are controlled by means of operating means not shown, which can be activated with suitable means on control panel 90. The difference between the line 15 and 16 is that about the line 15 a heating element 17 is placed which can be controlled in the desired way. The means necessary for that are also not shown, but they will be obvious for the expert. A detergent supply line 21 which receives a detergent means from the detergent reservoir 20 debouches into line 15. An adjustable meter 22 is located between the detergent line 21 and the water mains 15, and is also controlled by means not shown. A steam source 5 is accommodated on the right side in the cupboard 3 which can be filled via supply line 11 and closable line 13 with distilled or demineralized water. A discharge line for steam 25 extends from the steam source 5. In addition there is a compressor 6 or a connection for an external compressor in the cupboard 3, which is connected to a compressed air line 23.

The water line 16 and the water/detergent line 15 rejoin and then terminate at a multi-way valve 26, where the compressed air line 23 and the steam line 25 also end. On the downstream side of the multi-way valve 26 there is a discharge line 28, which leads to the connecting coupling A1 and in which a valve 50 can be effective. In the connecting coupling A1 the line 28 branches off into three lines 29.1, 29.2 and 29. 3, ...., 29.n. These lines 29.1, 29.2 29.3, ......, 29.n. are arranged in such a way with regard to one another that after having been connected to a connecting coupling A1 of the rear of the grip portion of a pneumatically driven angle piece 30, while sealed, they are aligned with passages or lumen in the angle piece. These passages are shown in figure 3, and are arranged in the interior of the angle piece. Compressed air line 31, return air line 32, spray air line 33 and spray or cooling water line 34 can be distinguished. The return air line 32 is, moreover, aligned with passage 29.4 in connecting coupling A1, which passage connects into line 27 which leads to a receptacle 9, which in turn can be connected to a discharge line X.

It will be understood that the remaining connecting couplings A2, A3, ...... An, will be similarly embodied, the line portions 28 always being sufficiently branched to ensure passage to the respective connecting couplings. Couplings for pneumatic/mechanical and electric/mechanical angle pieces are somewhat differently executed because of the desired drive of the pneumatic/mechanical and electric/mechanical angle pieces, in particular because of the spindle thereof. For a better understanding a cross section of a suchlike angle piece is shown in figure 4, the wall 40 defining a lumen 42, in which the spindle 41 is located. Spray air line 43 and spray water line 44 are further arranged in the wall 40. The connecting coupling A4 will therefore have to have a line connection to lumen 42 and to the lines 43 and 44, and furthermore a mechanically driven coupling for spindle 41, so as to allow the spindle to rotate during the cleaning and disinfecting for increasing the effectiveness of the cleaning/disinfecting.

If pneumatically driven angle piece 30 has to be cleaned the latter is coupled with its rear to connecting coupling A1. The fluid flow which will follow the route of the drive air will, in addition, take care of the drive during the cleaning.

Assuming that the reservoir 4 and the steam source 5, as well as detergent reservoir 20 are sufficiently filled, the apparatus 1 will be activated. The user can do this by operating the desired buttons on a control panel 90 by which a previously installed programme is possibly started up. An internal accumulator or battery or an external supply source, via an electric cable, can be used for the energy needed.

The pump 100 is activated and will propel the water from the reservoir 4 through line 14. The valve 19 will be closed so that the water flows through line 15 and whilst flowing is heated by heating unit 17. Detergent is supplied by supply means 22. For example as a detergent an all purpose cleaner which satisfies the requirements made above can be used and can be added in a concentration of e.g. 1 to 30. The detergent solution will then flow to the multi-way valve 26, which is adjusted such that the solution can flow directly through to passage 28. The detergent solution then enters the lines 31, 33 and 34 via the lines 29.1-29.3 and comes back via the angle piece head via the line 32. Surplus soap solution disappears between cracks and chinks in the angle piece head and is collected in the bowl 7, which can discharge to receptacle 9. The returned flow of detergent solution in which loosened lubricating oil and impurities are included is also lead to receptacle 9 via connection 29.4 and line 27, to be removed from there later or to be directly discharged.

During the washing process the heating unit 17 is operated in such a way that in approximately 3 minutes the temperature of the water rises in from 20° to at least 60°C. For increasing the cleaning effect the pump 100 can be controlled such that a pulsating flow of detergent solution is produced, with for example 10 seconds flow/10 seconds rest, and so on. Subsequently water is kept going for two minutes at that temperature with detergent solution dissolved therein. Therefore a start is made with cold washing, and gradually switching to warm washing.

After washing valve 18 is closed and valve 19 is opened so that water pumped by pump 100 will flow through line 16, and detergent free water arrives at multi-way valve 26 and is transferred to angle piece 30. During this flushing soap residue and impurities are discharged from angle piece 30.

Immediately after flushing the multi-way valve 26 is automatically switched to form a connection between steam supply line 25 and the passage 28. As a result steam is supplied under pressure to the angle piece and a wet disinfection by saturated steam injection takes place, as it were, without use being made of chemical means, and nontheless at a temperature which is lower than the temperature (121°C) at which sterilization normally takes place. The temperature of the introduced steam is, moreover, such that the flow of steam leaving the piece of equipment will have a temperature of at least approximately 100°C. Because the hollow spaces are precleaned the steam is an effective disinfection means. It is advantageous if care has been taken that the cross section of the return line is smaller than the cross section of the supply air line, in view of the fact that the steam is then forced to escape at the turbine in the angle piece head and can carry out its disinfecting action in all cracks and chinks. In doing so it is ensured that the number of revolutions is kept within the limits indicated by the manufacturer.

After steaming the multi-way valve 26 is automatically switched over so as to connect the compressed air line 23 to the passage 28. Relatively cool compressed air is then forced out of the compressor 6 via a microfilter to the angle piece 30, for removing condensed steam and for cooling the angle piece so that the latter is soon ready for use again by the dentist.

In this way an angle piece can be cleaned within a quarter of an hour in such a way that the latter can be used for the next patient without the risk of cross-infection.

In an alternative way washing can be carried out such that the angle pieces, after they have been mounted to the connecting couplings, are submerged in water. For this purpose the bowl 7 will have to extend to well above the level of the connecting couplings, in the case of the apparatus of figure 1.

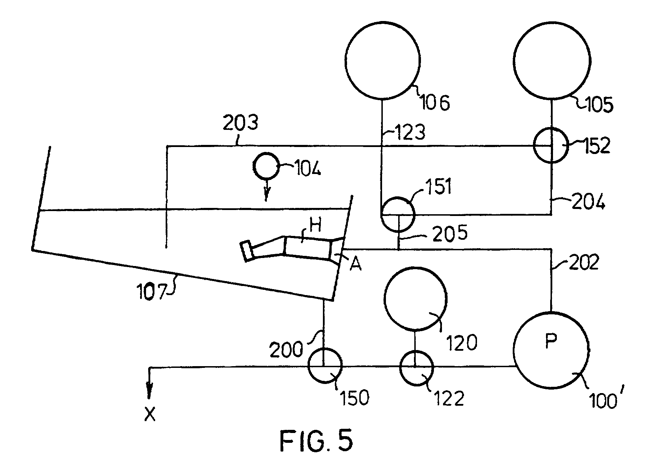

In figure 5 the arrangement needed for this is shown in a schematic way. Herewith operation can take place as follows. In bowl 107 the angle piece H is connected to connecting coupling A. The bowl 107 is filled with unheated water from reservoir 104, until the angle piece H is completely submerged. Then the pump 100' is operated to circulate water from the bowl 107 via line 200, two-way valve 150, line 201, pump 100', line 202 and angle piece H. Furthermore, detergent is supplied from detergent reservoir 120, via meter 122, to the water so that a detergent solution is present in the bowl 107. Subsequently, whilst continuously yet in a pulsating way pumping (10 seconds on, 10 seconds off) with pump 100' steam is fed into the detergent solution in the bowl 107 from steam source 105, via line 125, two-way valve 152 and line 203, the end of which is located below the level of the detergent solution in the bowl 107. The solution in the bowl is thereby gradually, for example in 3 minutes, heated to 60°-70°C, after which the supply of steam is stopped. The detergent solution is then circulated at that temperature for a few minutes (for example 2 minutes) by the pump in a pulsating way, via angle piece H. Then the pump is turned off and the two-way valve 150 is adjusted to connect line 200 with discharge X. Then the valve 150 is adjusted again and fresh water is drawn from reservoir 104 and circulated via angle pieces for flushing. The flush water can be discharged after a time (30 seconds) by yet again switching the valve 150. Alternatively water could be supplied upstream (not shown) from angle piece H to achieve a maximum flushing effect, the valve 150 then connecting line 200 with discharge X. After the flushing water has been discharged the bowl is refilled with water until the level above the angle piece. Then the steam source 105 is reactivated and the valve 152 is adjusted to connect the line 125 to line 204, and the valve 151 is set so that the line 204 connects with the line 205 and thus with line 202. As a result steam can be forced through the angle piece H, which can take place in a pulsating way. The temperature of the angle piece is prevented from getting too high by the water bath of the angle piece which also prevents the steam from evaporating after deposit on the inner surface, thus not being able to carry out its disinfecting action. The concentrated steam is deposited via line 200 and discharge X. After the steam treatment the steam source 105 is closed off and the valve 151 is adjusted in order to connect the line 123 of compressed air stream 106 with line 205, so that drying and cooling off of the angle piece H can take place.

The apparatus 1 of figure 1 is also suitable for cleaning with a view to removing biofilm and other impurities in hollow spaces, in particular in hoses. For this purpose the hose is, on the one hand, connected to a connecting coupling S₁-Sₙ and, on the other hand, to the entrances R₁-Rₙ, possibly via a suitable adapter, and then subjected to the cleaning process described above. The washing phase can, alternatively, be omitted.

Other medical and paramedical apparatuses (endoscopes) in which there are lumina which can become contaminated with micro-organisms can be cleaned and disinfected with the apparatus 1 by using suitable connecting provisions.

Possibly, a reservoir and supply lines for lubricating oil can be added on to the apparatus 3, which also open into multi-way valve 26, and/or decalcifying means. If only a few of the connecting couplings A1-An are being used, the connecting couplings not in use are closed by means of their pertaining valve 50.

The processing time depends on the length of the hoses/lines, and the composition of the unit, which can be different for each unit.

The operating means which can be activated by means of the operating of the control panel 90, can be programmable for expert personnel, preferably beforehand. In this way it is achieved that the process can take place automatically without there being the risk that due to a wrong action of the doctor or his assistant the cleaning/disinfecting process is carried out in an incorrect manner.

The lines and most of the other passages in the apparatus 1 are made of metal or a synthetic (teflon) with a small biofilm forming capacity. The apparatus can further be provided with additional bypass lines (not shown) in order to permit flushing of the lines and passages for cleaning them.

## Claims

1. Method for cleaning hollow spaces in dental equipment having driven parts, in particular hand and angle pieces, **characterized in that** a flow of steam is forced through the hollow spaces, and **in that** prior to the flow of steam a flow of detergent solution is forced through the hollow spaces.

2. Method according to claim 1, wherein after the flow of detergent solution and prior to the flow of steam a flow of flushing water is forced through the hollow spaces.

3. Method according to claim 1 or 2, wherein the detergent solution is heated at least for a time, wherein preferably the temperature of the detergent solution during the flow through is gradually increased to a minimum of approximately 60°C, preferably during at least of about 3 minutes, and wherein during at least a part of the period of flow through with detergent solution the latter is at a minimum of approximately 60°C, preferably during at least about 2 minutes.

4. Method according to claim 3, wherein an unheated detergent solution is forced through the hollow spaces prior to the heating.

5. Method according to claim 3, wherein the detergent solution is heated with the help of steam.

6. Method according to any one of the 2-5, wherein the detergent solution is passed through in a pulsating way.

7. Method according to any one of the preceding claims, wherein the flow of steam is passed through in a pulsating way.

8. Method according to any one of the preceding claims, wherein the period in which steam flows through lasts a minimum of approximately 5 minutes.

9. Method according to any one of the preceding claims, wherein during the flow through with steam the equipment is kept immersed in water.

10. Method according to any one of the preceding claims, wherein in case angle pieces or hand pieces are cleaned, the rotatable parts thereof are driven during the cleaning.

11. Method according to any one of the preceding claims, wherein after the flow through with steam, flow through with preferably cooled, compressed air which is free of micro-organisms takes place.

12. Method according to any one of the preceding claims, wherein after passage through the piece of equipment concerned at least a part of the steam supplied for the flow through is discharged via lines directly connected to the piece of equipment.

13. Apparatus (1) for cleaning the hollow spaces of driven pieces of dental equipment, in particular hand and angle pieces, comprising at least one first connecting coupling (A1) for the piece of equipment which is accessible from the outside and which is provided with at least one passage (29.1-29.4) for fluid, **characterized by** means (5) for the production and passage of steam, which by means of first line means (25) are in fluid communication with said first connecting coupling (A1), further comprising second line means (15,16) for the passage of water, which are in fluid communication with said at least one coupling (A1), and third line means (21) for detergents, which are connected to the second line means (15) by means of controllable detergent discharge means (22), pumping means (100) for urging the water to the first connecting coupling (A1), and controllable valve means (26) for selectively bringing the first, second and third line means into fluid communication with the fluid passage in the connecting coupling, further comprising controllable means (17) for heating the water in the second line means (15).

14. Apparatus according to claim 13, further comprising connecting means (10,12) for connecting the second line means (15,16) to a water source located outside the apparatus (1), such as the water main.

15. Apparatus according to claim 13 or 14, further comprising a first reservoir (4) for water, which is provided with filling means, preferably further comprising fourth line means for the passage of water from the water reservoir to the steam source (5).

16. Apparatus according to claim 15, further comprising a second reservoir for demineralized or distilled water intended for the production of steam.

17. Apparatus according to any one of the claims 13-16, wherein the second line means have two parallel portions (15,16), the first (15) of which is influenced by the heating means (17) and wherein valve means (18,19) are provided for selectively closing the first or the second parallel portion of the second line means.

18. Apparatus according to any one of the claims 13-17, further comprising a compressed air source (6) and fifth line means (23) for fluid communication of the compressed air source with the valve means (29.1-29.4) for the connecting coupling (A1).

19. Apparatus according to any one of the claims 13-18, comprising a number of first connecting couplings (A1-An) for hand and angle pieces, wherein at least one of the first connecting couplings is provided with means for driving a mechanically driven angle or hand piece.

20. Apparatus according to any one of the claims 13-19, wherein the first connecting coupling(s) (A1-An) is (are) accommodated in a closable, cupboard-shaped room.

21. Apparatus according to any one of the claims 13-20, further comprising a number of second connecting couplings (S1-Sn) for hoses of hand and angle pieces, sprayers and so on and/or for a hose for connection to a so-called dental treating unit, and preferably further comprising a connecting coupling (R1-Rn) for the receipt of fluids passed through treated hoses, which coupling is in fluid connection with a receptacle (9).

22. Apparatus according to claim 20 or 21, wherein the connecting coupling(s) (R1-Rn, S1-Sn) is(are) provided with several fluid passages, which are selectively closable with the help of valve means.

23. Apparatus according to any one of the claims 13-22, provided with wheels (24) for making it mobile.

24. Apparatus according to any one of the claims 13-23, comprising adjustable, preferably programmable control means for said parts, such as the pump (100), the compressor (6), the steam source (5) and the valves (12, 13, 18, 19, 22, 26).

25. Assembly comprising an apparatus according to claim 21 and a dental treating unit, wherein the unit is provided with a connecting coupling for a fluid hose which extends between the apparatus (1) and the unit.

## Patentansprüche

1. Verfahren zum Reiningen von Hohlräumen in zahnärztlicher Apparatur mit angetriebenen Teilen, insbesondere Hand- und Winkelstücken, **dadurch gekennzeichnet, daß** ein Dampfstrom durch die Hohlräume gezwungen wird, und daß vor dem Dampfstrom ein Strom Detergensauflösung durch die Hohlräume gezwungen wird.

2. Verfahren nach Anspruch 1, wobei nach dem Strom Detergensauflösung und vor dem Dampfstrom ein Strom Spülwasser durch die Hohlräume gezwungen wird.

3. Verfahren nach Anspruch 1 oder 2, wobei die Detergensauflösung zumindest eine Weile erhitzt wird, wobei vorzugsweise die Temperatur der Detergensauflösung während dem Durchströmen allmählich bis minimal ungefähr 60°C erhöht wird, vorzugsweise während zumindest ungefähr 3 Minuten, und wobei während zumindest einem Teil der Periode von Durchströmen mit Detergensauflösung diese Letzte minimal ungefähr 60°C ist, vorzugsweise während zumindest ungefähr 2 Minuten.

4. Verfahren nach Anspruch 3, wobei eine ungeheizte Detergensauflösung vor dem Erhitzen durch die Hohlräume gezwungen wird.

5. Verfahren nach Anspruch 3, wobei die Detergensauflösung mit Hilfe von Dampf erhitzt wird.

6. Verfahren nach einem der Ansprüche 2-5, wobei die Detergensauflösung pulsierend durchgeführt wird.

7. Verfahren nach einem der vorhergehenden Ansprüche, wobei der Dampfstrom pulsierend durchgeführt wird.

8. Verfahren nach einem der vorhergehenden Ansprüche, wobei die Periode worin Dampf durchströmt minimal ungefähr 5 Minuten dauert.

9. Verfahren nach einem der vorhergehenden Ansprüche, wobei während dem Durchströmen mit Dampf, die Apparatur in Wasser eingetaucht bleibt.

10. Verfahren nach einem der vorhergehenden Ansprüche, wobei falls Winkel- oder Handstücke gereinigt werden, deren Drehteile während der Reinigung angetrieben werden.

11. Verfahren nach einem der vorhergehenden Ansprüche, wobei nach dem Durchströmen mit Dampf, Durchströmung mit vorzugsweise gekühlter, mikroorganismusfreier Preßluft stattfindet.

12. Verfahren nach einem der vorhergehenden Ansprüche, wobei nach Durchführung durch die betreffende Apparatur, zumindest ein Teil des für die Strömung zugeführten Dampfes via direkt mit der betreffenden Apparatur verbundene Leitungen abgeführt wird.

13. Vorrichtung (1) zum Reinigen von Hohlräumen von angetrieben Teilen von zahnärtzlicher Apparatur, insbesondere Hand- und Winkelstücken, umfassend zumindest eine erste Anschlußkupplung (A1) für das Teil der Apparatur, die von außen zugänglich ist und die mit zumindest einem Durchgang (29.1-29.4) für Flüssigkeit versehen ist, **gekennzeichnet durch** Mittel (5) für die Produktion und Durchführung von Dampf, die **durch** erste Leitungsmittel (25) in flüssiger Verbindung mit der ersten Anschlußkupplung (A1) stehen, weiter umfassend zweite Leitungsmittel (15, 16) für die Durchführung von Wasser, die in flüssiger Verbindung mit der zumindest einen Kupplung (A1) stehen, und dritte Leitungsmittel (21) für Reinigungsmittel, die **durch** regulierbare Detergensabgabemittel (22) mit den zweiten Leitungsmitteln (15) in Verbindung stehen, Pumpmittel (100) zum zu der ersten Anschlußkupplung (A1) Zwingen von Wasser, und regulierbare Ventilmittel (26) um die ersten, zweiten und dritten Leitungsmittel selektiv in flüssiger Verbindung mit dem Flüssigkeitsdurchgang in der Anschlußkupplung zu bringen, weiter umfassend regulierbare Mittel (17) zum Erhitzen von Wasser in den zweiten Leitungsmitteln (15).

14. Vorrichtung nach Anspruch 13, weiter umfassend Verbindungsmittel (10, 12) zur Verbindung der zweiten Leitungsmittel (15, 16) mit einer außer der Vorrichtung (1) gelegenen Wasserquelle, wie der Wasserleitung.

15. Vorrichtung nach Anspruch 13 oder 14, weiter umfassend ein erstes Reservoir (4) für Wasser, das mit Füllmitteln versehen ist, vorzugsweise weiter umfassend vierte Leitungsmittel für die Durchführung von Wasser von dem Wasserreservoir zu der Dampfquelle (5).

16. Vorrichtung nach Anspruch 15, weiter umfassend ein zweites Reservoir für für Dampfbildung bestimmtes, entmineralisiertes oder destilliertes Wasser.

17. Vorrichtung nach einem der Ansprüche 13-16, wobei die zweiten Leitungsmittel zwei parallele Abschnitte (15, 16) haben, wovon der erste (15) durch die Heizmittel (17) beeinflusst wird, und wobei Ventilmittel (18, 19) vorgesehen sind, zum selektiv Abschließen des ersten oder des zweiten parallelen Abschnitts der zweiten Leitungsmittel.

18. Vorrichtung nach einem der Ansprüche 13-17, weiter umfassend eine Preßluftquelle (6) und fünfte Leitungsmittel (23) zur flüssiger Verbindung der Luftpreßquelle mit den Ventilmitteln (29.1-29.4) für die Anschlußkupplung (A1).

19. Vorrichtung nach einem der Ansprüche 13-18, umfassend eine Anzahl erste Anschlußkupplungen (A1-An) für Hand- und Winkelstücke, wobei zumindest eine der ersten Anschlußkupplungen mit Mitteln zum Antrieb eines mechanisch angetriebenen Winkel- oder Handstückes versehen ist.

20. Vorrichtung nach einem der Ansprüche 13-19, wobei die erste(n) Anschlußkupplung(en) in einem abschließbaren, schrankförmigen Raum untergebracht ist (sind).

21. Vorrichtung nach einem der Ansprüche 13-20, weiter umfassend eine Anzahl zweite Anschlußkupplungen (S1-Sn) für Schläuche von Hand- und Winkelstücken, Spritzdüsen und so weiter, und/oder für einen Schlauch zur Verbindung mit einer sogenannten zahnärtzlichen Behandlungseinheit, und vorzugweise weiter umfassend eine Anschlußkupplung (R1-Rn) für den Empfang von durch behandelte Schläuche geführten Flüssigkeiten, welche Kupplung in flüssiger Verbindung mit einem Auffangbehälter (9) steht.

22. Vorrichtung nach Anspruch 20 oder 21, wobei die Anschlußkupplung(en) (R1-Rn, S1-Sn) mit verschiedenen flüssigen Durchgängen versehen ist (sind), die mit Hilfe von Ventilmitteln selektiv abschließbar sind.

23. Vorrichtung nach einem der Ansprüche 13-22, mit Rädern (24) zum fahrbar machen davon versehen.

24. Vorrichtung nach einem der Ansprüche 13-23, umfassend regulierbare, vorzugsweise programmierbare Steuermittel für die genannten Teile, wie die Pumpe (100), den Kompressor (6), die Dampfquelle (5) und die Ventile (12, 13, 18, 19, 22, 26).

25. System umfassend eine Vorrichtung nach Anspruch 21, und eine zahnärtzliche Behandlungseinheit, wobei die Einheit mit einer Anschlußkupplung für einen sich zwischen der Vorrichtung (1) und der Einheit erstreckenden Schlauch für Flüssigkeit versehen ist.

## Revendications

1. Procédé de nettoyage d'espaces creux dans un équipement de dentisterie comportant des pièces entraînées, en particulier des pièces d'angle et à main, **caractérisé en ce que** de la vapeur est injectée dans les espaces creux, et **en ce qu'**avant l'injection de vapeur, une solution détergente est injectée dans les espaces creux.

2. Procédé selon la revendication 1, dans lequel après l'injection de la solution détergente et avant l'injection de vapeur de l'eau de lavage est injectée dans les espaces creux.

3. Procédé selon la revendication 1 ou 2, dans lequel la solution détergente est chauffée pendant un certain temps, dans lequel de préférence la température de la solution détergente pendant l'injection est progressivement augmentée au moins jusqu'à 60°C approximativement, de préférence pendant au moins 3 minutes environ, et dans lequel pendant au moins une partie de la période d'injection de la solution détergente cette dernière est à une température d'au moins 60°C approximativement, de préférence pendant au moins 2 minutes environ.

4. Procédé selon la revendication 3, dans lequel une solution détergente non chauffée est injectée dans les espaces creux avant le chauffage.

5. Procédé selon la revendication 3, dans lequel la solution détergente est chauffée à l'aide de la vapeur.

6. Procédé selon l'une quelconque des revendications 2 à 5, dans lequel l'injection de la solution détergente est pulsée.

7. Procédé selon l'une quelconque des revendications précédentes, dans lequel l'injection de vapeur est pulsée.

8. Procédé selon l'une quelconque des revendications précédentes, dans lequel la période d'injection de la vapeur dure au moins 5 minutes approximativement.

9. Procédé selon l'une quelconque des revendications précédentes, dans lequel l'équipement est maintenu immergé dans l'eau pendant l'injection de la vapeur.

10. Procédé selon l'une quelconque des revendications précédentes, dans lequel, dans le cas de nettoyage de pièces d'angle ou de pièces à main, les parties rotatives de celles-ci sont entraînées pendant le nettoyage.

11. Procédé selon l'une quelconque des revendications précédentes, dans lequel, après l'injection de vapeur, il est injecté de l'air comprimé, de préférence refroidi, qui est dépourvu de microorganismes.

12. Procédé selon l'une quelconque des revendications précédentes, dans lequel au moins une partie de 1a vapeur injectée dans la pièce d'équipement en question est ensuite évacuée par des conduits reliés directement à la pièce d'équipement.

13. Appareil (1) de nettoyage des espaces creux de pièces entraînées d'un équipement de dentisterie, en particulier des pièces à main et d'angle, comprenant au moins un premier raccord (A1) destiné à la pièce d'équipement qui est accessible de l'extérieur et qui est doté d'au moins un passage (29.1 à 29.4) pour le fluide, **caractérisé par** des moyens (5) pour la production et le passage de vapeur, qui à l'aide de premiers moyens formant conduit (25) sont en communication fluidique avec ledit premier raccord (15, 16), comprenant en outre des deuxièmes moyens formant conduit (15, 16) pour le passage de l'eau, qui sont en communication fluidique avec ledit au moins un raccord (A1), et des troisièmes moyens formant conduit (21) pour les détergents, qui sont reliés aux seconds moyens formant conduit (15) à l'aide de moyens commandés d'évacuation de détergent (22), des moyens de pompage (100) pour pousser l'eau vers le premier raccord (A1), et des moyens commandés formant clapet (26) pour amener sélectivement les premiers, deuxièmes et troisièmes moyens formant conduits en communication fluidique avec le passage de fluide dans le raccord, comprenant en outre des moyens commandés (17) pour chauffer l'eau dans les deuxièmes moyens formant conduit (15).

14. Appareil selon la revendication 13, comprenant en outre des moyens de raccordement (10, 12) pour raccorder les deuxièmes moyens formant conduit (15, 16) à une source d'eau placée à l'extérieur de l'appareil (1), tel que le réseau d'alimentation en eau.

15. Appareil selon la revendication 13 ou 14, comprenant en outre un premier réservoir (4) pour l'eau, qui est doté de moyens de remplissage, comprenant en outre de préférence des quatrièmes moyens formant conduit pour le passage de l'eau du réservoir d'eau à la source de vapeur (5).

16. Appareil selon la revendication 15, comprenant en outre un second réservoir pour l'eau déminéralisée ou l'eau distillée prévue pour produire de la vapeur.

17. Appareil selon l'une quelconque des revendications 13 à 16, dans lequel les deuxièmes moyens formant conduit comportent deux portions parallèles (15, 16), dont la première (15) est sous l'influence de moyens chauffants (17) et dans lequel des moyens formant clapets (18, 19) sont prévus pour fermer sélectivement la première portion ou la seconde portion parallèle des deuxièmes moyens formant conduit.

18. Appareil selon l'une quelconque des revendications 13 à 17, comprenant en outre une source d'air comprimé (6) et des cinquièmes moyens formant conduit (23) pour la communication fluidique de la source d'air comprimé avec les moyens formant clapets (29.1 à 29.4) pour le raccord (A1).

19. Appareil selon l'une quelconque des revendications 13 à 18, comprenant un certain nombre de premiers raccords (A1 à An) pour des pièces à main ou d'angle, dans lequel au moins un des premiers raccords est doté de moyens pour entraîner mécaniquement une pièce à main ou d'angle entraînée.

20. Appareil selon l'une quelconque des revendications 13 à 19, dans lequel le ou les premiers raccords (A1 à An) sont logés dans un compartiment de type armoire pouvant être fermé.

21. Appareil selon l'une quelconque des revendications 13 à 20, comprenant en outre un certain nombre de deuxièmes raccords (Si à Sn) pour tuyaux de pièces à main ou d'angle, de pulvérisateurs etc. et/ou pour un tuyau de raccordement à ce que l'on appelle une unité de soin dentaire, et de préférence comprenant en outre un raccord (R1 à Rn) pour recevoir des liquides traversant des tuyaux traités, lequel raccord est en communication fluidique avec un récipient (9).

22. Appareil selon la revendication 20 ou 21, dans lequel le ou les raccords (R1 à Rn, S1 à Sn) sont dotés de plusieurs passages de fluide, qui peuvent être fermés sélectivement à l'aide de moyens formant clapets.

23. Appareil selon l'une quelconque des revendications 13 à 22, doté de roues (24) pour le rendre mobile.

24. Appareil selon l'une quelconque des revendications 13 à 23, comprenant des moyens de commande réglables, de préférence programmables, pour lesdites pièces, telles que la pompe (100), le compresseur (6), la source de vapeur (5) et les clapets (12, 13, 18, 19, 22, 26).

25. Ensemble comprenant un appareil selon la revendication 21 et une unité de soin dentaire, dans lequel l'unité est dotée d'un raccord pour un tuyau de fluide qui s'étend entre l'appareil (1) et l'unité.
